# EUROPÄISCHE PATENTSCHRIFT

(11) **EP 2 934 421 B1**
(45) Veröffentlichungstag und Bekanntmachung des Hinweises auf die Patenterteilung: **04.10.2017**
(21) Anmeldenummer: 13814059.5
(22) Anmeldetag: 03.12.2013
(51) Int. Cl.: A61J 1/14, A61M 39/16, B65D 47/06, B65D 51/16

(54) **MILCHFLASCHENADAPTER**
MILK BOTTLE ADAPTER
ADAPTATEUR POUR BOUTEILLE DE LAIT

(30) Priorität: 21.12.2012 US 201261740499 P; 21.12.2012 EP 12199006
(43) Veröffentlichungstag der Anmeldung: 28.10.2015
(73) Patentinhaber: Fresenius Kabi Deutschland GmbH, 61352 Bad Homburg (DE)
(72) Erfinder: BECKER, Michael, 75438 Knittlingen (DE); BREUER-THAL, Barbara, 65510 Idstein (DE); ALTHAUS-KROHN, Sabine, 61381 Friedrichsdorf (DE)
(74) Vertreter: Kusche, Robert
(86) Internationale Anmeldenummer: PCT/EP2013/075390
(87) Internationale Veröffentlichungsnummer: WO 2014/095351

(56) Entgegenhaltungen:
- EP-A1- 2 436 421
- WO-A1-99/32168
- WO-A1-99/61093
- WO-A1-99/61093
- DE-A1-102009 044 319
- FR-A1- 2 761 665
- GB-A- 797 812
- GB-A- 797 812
- GB-A- 2 337 035
- US-A- 5 637 107
- US-A- 5 992 701
- US-A1- 2010 084 397

## Beschreibung

Die Erfindung betrifft eine Kappe gemäß dem Oberbegriff des Anspruchs 1. Die Erfindung betrifft ferner eine Milchflasche und ein Set umfassend eine solche Kappe.

Erfindungsgemäße Kappen werden dazu verwendet, um ein Rohr, wie eine Spitze einer Spritze anzuschließen, so dass eine Leitung entsteht, über die eine Übertragung eines Mediums durch das Rohr und die Kappe erfolgen kann.

In der Neonatologie und Pädiatrie wird, wenn es der Entwicklungszustand des gastrointestinalen Traktes beim Frühgeborenen zulässt, beziehungsweise wenn eine Saugschwäche bei Kleinkindern vorliegt, flüssige Nahrung über Ernährungssonden verabreicht. Wenn die Möglichkeit besteht, das heißt die Mutter in der Lage ist, über beispielsweise eine Milchpumpe Muttermilch in einen Milchbehälter, insbesondere in eine Milchflasche, abzupumpen, wird bevorzugt Muttermilch als flüssige Nahrung verabreicht.

Ein Milchbehälter im Sinne der vorliegenden Erfindung ist ein Lagerbehälter mit starren oder flexiblen Wänden, der zur Aufbewahrung von Muttermilch geeignet ist, so dass diese anschließend zur Ernährung von Kleinkindern, wie Babys und Frühgeborenen, geeignet ist und verwendet werden kann. Der Milchbehälter muss also aus geeigneten Materialien bestehen, um die Flüssigkeit (die Muttermilch) zu halten und die Flüssigkeit nicht durch Schadstoffe oder Mikroorganismen zu verunreinigen und damit unbrauchbar zu machen. Ein solcher Milchbehälter umfasst dazu eine Öffnung, über die die Muttermilch eingeführt werden kann. Über diese Öffnung oder eine andere Öffnung kann die Muttermilch auch entnommen werden. Die Milchbehälter verfügen meist über einen Weithals, der beispielsweise mit einem Sauger, dem Ende einer Pumpeinrichtung und/oder einem Deckel verbunden werden kann. Typische Milchbehälter sind also beispielsweise handelsübliche Milchflaschen, wie z.B. Babyflaschen, mit einem Weithalsgewinde. Es können aber auch Beutel als Milchbehälter verwendet werden. Die Muttermilch aus der Brust der Mutter des Frühgeborenen wird in einen Milchbehälter gepumpt und gegebenenfalls vorher aufbereitet.

Zur enteralen Versorgung von Frühgeborenen wird die Muttermilch in der Frühgeborenen-Station oder der Milchküche des Krankenhauses vorbereitet und mittels einer Spritze aus dem Milchbehälter entnommen. Abhängig von der körperlichen Entwicklung des Frühgeborenen wird die entnommene Muttermilch zum Beispiel durch eine nasogastrale Ernährungssonde verabreicht. Da die Mägen dieser Patienten sehr klein sind (in etwa 3 cm im Durchmesser) muss die Ernährung in sehr kleinen Portionen erfolgen (durchschnittlich etwa 7,5 ml pro Spritze). Deswegen müssen bis zu zwölf Spritzen mit Nahrung für jedes Frühgeborene pro Tag vorbereitet werden.

Die Muttermilch wird derzeit aus dem Milchbehälter entnommen, indem die Spitze einer Spritze in die Flüssigkeit eingetaucht und die Flüssigkeit anschließend mit der Spritze aufgezogen wird.

Nachteilig ist hieran, dass diese Vorgehensweise aufwendig und umständlich in der Handhabung ist. Zudem besteht das Risiko eines Verlusts eines Teils der Muttermilch beim Aufziehen selbst. Weiterhin kann der Verbleib einer ungenutzten Restmenge in dem Milchbehälter nicht ausgeschlossen werden. Des Weiteren kann über die Öffnung des Milchbehälters die Muttermilch kontaminiert werden. Ferner kostet die gesamte Vorgehensweise viel Zeit und ist umständlich, was im oft hektischen Krankenhausbetrieb besonders nachteilig ist.

Eine Kappe der eingangs genannten Art ist aus der US2010/084397A1 bekannt.

Aufgabe der Erfindung ist es, diese und weitere nicht genannte Nachteile zu überwinden. Für die Entwicklung frühgeborener Kinder ist Muttermilch die wertvollste Nahrung. Daher ist es sehr wichtig und insbesondere Aufgabe der vorliegenden Erfindung eine Vorrichtung zur Verfügung zu stellen, bei dem die Muttermilch möglichst nicht kontaminiert wird und bei dem der Verlust von Muttermilch möglichst vermieden wird. Zudem soll möglichst die gesamte Muttermilch nutzbar sein. Eine einfache Bedienbarkeit und eine sichere Bereitstellung durch das Krankenhauspersonal ist ebenso Aufgabe der vorliegenden Erfindung. Gleichzeitig soll die Vorrichtung einfach und kostengünstig aufgebaut sein.

Diese Aufgaben werden bei einer Kappe der eingangs genannten Art durch die kennzeichnenden Merkmale des Anspruchs 1 gelöst. Das weibliche Konnektorelement umfasst ein Rohr, welches zumindest an einem Abschnitt der Rohrinnenseite die antimikrobielle Oberfläche aufweist, welche durch eine antimikrobielle Beschichtung bereitgestellt ist. Die Flüssigkeit ist insbesondere eine Flüssigkeit für die enterale Ernährung, beispielsweise Milch.

Bei einer solchen erfindungsgemäßen Kappe kann das männliche Konnektorelement eine Spitze einer Spritze sein. Die Spitze einer Spritze oder ein anderes Verbindungselement kann bei erfindungsgemäßen Kappen in das weibliche Konnektorelement aufgenommen werden, um eine sichere und keimfreie Verbindung zwischen einem angeschlossenen Milchbehälter und der Spritze zu bilden. Es wird vorzugsweise eine fluiddichte Verbindung bereitgestellt, die ein Durchleiten einer Flüssigkeit durch die Kappe ermöglicht.

Die Kappe kann mit der Spritze lösbar verbunden sein. Unter einer antimikrobiellen Oberfläche wird im Rahmen der vorliegenden Erfindung eine Oberfläche verstanden, die eine antimikrobielle Substanz , enthält oder aus dieser besteht, die insbesondere die Vermehrungsfähigkeit oder Effektivität von Mikroorganismen reduziert oder Mikroorganismen abtötet beziehungsweise inaktiviert.

Dabei kann vorgesehen sein, dass die Halterung ein Weithalsflaschenadapter, ein Schmalhalsflaschenadapter und/oder eine Steckverbindung ist, wobei die Halterung vorzugsweise mehrere Anschlussmöglichkeiten umfasst.

Bei erfindungsgemäßen Kappen mit Weithalsflaschenadapter kann vorgesehen sein, dass der Weithalsflaschenadapter einen Durchmesser von 2 cm bis 10 cm, bevorzugt mit einem Durchmesser von 3 cm bis 8 cm, besonders bevorzugt mit einem Durchmesser von 4 cm bis 6 cm hat.

Bei der Verwendung von Weithalsflaschenadaptern als Halterung können auch handelsübliche Milchflaschen, insbesondere Babyflaschen, als Michbehälter verwendet werden. Das hat den Vorteil, dass keine getrennte Lagerhaltung notwendig ist. Variable Mehrzweckanschlüsse erlauben die Verwendung mit verschiedenen Milchbehältern.

Gemäß einer weiteren Ausgestaltung der Erfindung kann vorgesehen sein, dass die Kappe fluiddicht und/oder lösbar mit dem männlichen Konnektorelement verbindbar ist, wobei vorzugsweise der innere Mantel der Vertiefung, umlaufend und/oder flächig mit einem zumindest bereichsweise passenden männlichen Konnektorelementverbindbar ist. Dabei kann besonders bevorzugt vorgesehen sein, dass der innere Mantel der Vertiefung formschlüssig mit einer zumindest bereichsweise passenden Spitze der Spritze verbindbar ist. Es kann auch vorgesehen sein, dass der innere Mantel der Vertiefung umlaufend und flächig, vorzugsweise formschlüssig mit einer zumindest bereichsweise passenden Spitze der Spritze verbunden ist.

Die Dichtigkeit der Verbindung führt zu einer geringeren Gefahr des Verlusts von Muttermilch und zu einer geringeren Gefahr einer Kontamination der Muttermilch. Eine lösbare Spitze der Spritze macht eine Mehrfachverwendung möglich.

Erfindungsgemäß kann auch vorgesehen sein, das weibliche Konnektorelement zumindest abschnittsweise konisch ist und in dem weiblichen Konnektorelement ein zumindest abschnittsweise konisches, männliches Konnektorelement aufnahmbar ist. Vorzugsweise ist die Vertiefung in dem weiblichen Konnektorelement konisch und darin ein konisches, männliches Konnektorelement aufnehmbar. Es kann erfindungsgemäß auch vorgesehen sein, dass das männliche Konnektorelement in das weibliche Konnektorelement oder die Vertiefung aufgenommen oder darin befestigt, bevorzugt eingeschraubt ist. Die konische Form führt zu einer höheren Dichtigkeit der Verbindung, da ein konisches, männliches Konnektorelement kraftschlüssig in die Vertiefung eingesteckt werden kann.

In einer Ausgestaltung sind das weibliche Konnektorelement und/oder das männliche Konnektorelement ausgebildet als ein weibliches und/oder männliches Konnektorelement für die enterale und/oder gastrische Anwendung. In einer ersten Variante sind das weibliche und/oder männliche Konnektorelement als ein männliches und/oder weibliches PG-Lock-Konnektorelement ausgebildet. Das genannte PG-Lock-Verbindungssystem ist dem Fachmann bekannt und liegt der ISO Arbeitsgruppe ISO/CD 80369-3 "Enterale und gastrische Anwendungen" als Entwurf vor und ist dort einsehbar. in einer zweiten Variante sind das weibliche Konnektorelement und/oder das männliche Konnektorelement ausgebildet als ein weibliches und/oder männliches ENLock-Konnektorelement. Ein solches ENLockVerbindungssystem ist dem Fachmann bekannt und unter anderem in der WO 2010/115598 A 1 beschrieben. Ferner liegt das ENLock-Verbindungssystem als Entwurf der ISO Arbeitsgruppe - TC21 0 I JWG4, PG 3 - Enteral Connectors - vor und ist einsehbar. Der Inhalt der genannten Patentanmeldung wird vollumfänglich in die vorliegende Patentanmeldung durch Bezugnahme inkorporiert.

Das antimikrobielle Material ist beispielsweise vollständig oder abschnittsweise in das Material des weiblichen Konnektorelements eingebettet. Es kann vorgesehen sein, dass die antimikrobielle Oberfläche eine antimikrobielle Beschichtung der Oberfläche der Vertiefung oder der Öffnung ist. Beschichtungen ermöglichen, dass nur die betroffenen Oberflächen einen antimikrobiellen Wirkstoff enthalten müssen und diese nicht in dem gesamten Material enthalten sein müssen.

In einer Ausführungsform ist vorgesehen, dass die antimikrobielle Oberfläche Silber umfasst, vorzugsweise Silberpartikel, insbesondere mit einem mittleren Durchmesser von weniger als 1 µm.

Solche antimikrobiellen Oberflächen, beispielsweise mit Silber- oder Nano-Silber-Beschichtungen, stellen eine langlebige antimikrobielle Wirkung zur Verfügung, die den Einsatz von Desinfektionsmitteln reduzieren kann. Dadurch wird die Belastung des Immunsystems der Patienten reduziert und allergischen Reaktionen der Patienten vorgebeugt.

Gemäß einer Ausführungsform der Erfindung ist vorgesehen, dass die Vertiefung ein Innengewinde zum Befestigen eines männlichen Konnektorelements mit einem Außengewinde umfasst. Das Innengewinde und/oder das Außengewinden kann bzw. können durch einen einzigen Gewindegang bereitgestellt werden.

Erfindungsgemäße Kappen können sich auch dadurch auszeichnen, dass die Kappe einen fluiddichten Belüftungskanal umfasst, vorzugsweise umfassend ein Belüftungsventil und/oder einen Belüftungsfilter, insbesondere einen Bakterienfilter. Vorzugsweise weist bzw. weisen das Belüftungsventil und/oder der Belüftungsfilter zumindest bereichsweise eine hydrophobe Oberfläche auf. Durch diese Maßnahme werden auch Milchflaschen einsetzbar, die über keine flexiblen Wände verfügen. Der Druckausgleich erfolgt über den Belüftungskanal Durch die hydrophobe Oberfläche kann die Kappe auf einem Milchbehälter in einem Kühlschrank gelagert werden, ohne dass zu befürchten ist, dass nach dem Entfernen aus dem Kühlschrank der Filter durch kondensierendes Wasser aus der Luft (Luftfeuchtigkeit) unbrauchbar oder beeinträchtigt wird, beziehungsweise die Belüftung unterbrochen wird und so keine oder weniger Muttermilch in die Spritze gelangen kann.

Eine weitere Ausgestaltung der Erfindung kann vorsehen, dass die Kappe, insbesondere das weibliche Konnektorelement, eine Befestigungseinrichtung zur Befestigung des männlichen Konnektorelements umfasst. Vorzugsweise ist die Befestigungseinrichtung als ein Drehverriegelungselement, ein Bajonett-Verschluss oder ein Gewinde ausgeführt. Die Befestigungseinrichtung ist lösbar mit dem komplementären Konnektorelement verbindbar. Hierdurch wird eine stabile Verbindung der Spritze mit dem Milchbehälter möglich, so dass sich diese nicht ungewollt löst und die Gesundheit des Patienten gefährdet.

Gemäß einer weiteren Ausführungsform der Erfindung erstreckt sich das weibliche Konnektorelement vorzugsweise zentrisch durch die Kappe und/oder über eine Deckelfläche der Kappe hinaus. Es ist vorzugsweise durch einen Verschluss abdeckbar. Es kann vorgesehen sein, dass die Öffnung mit der Vertiefung in einem Rohr ausgebildet ist, das sich, insbesondere zentrisch, durch die Kappe erstreckt, wobei vorzugsweise zumindest auf der Seite zum Einführen des männlichen Konnektorelements das Rohr über eine Deckelfläche der Kappe übersteht und besonders bevorzugt durch einen Verschluss abdeckbar ist oder abgedeckt ist.

Durch das Rohr kann die Kontaktfläche der zylindrischen Vertiefung zur Spitze der Spritze besonders großflächig und dadurch stabil und dicht ausgeführt werden. Der "Deckel-Teil" mit der Deckelfläche der Kappe muss dann nicht besonders Dick ausgeführt werden, das heißt, er kann dünn (weniger als 4 mm dick) ausgeführt werden, um eine ausreichende Stabilität der Verbindung des männlichen Konnektorelements mit der Kappe zu erreichen.

Auch kann vorgesehen sein, dass die Kappe im Bereich der Öffnung, insbesondere das weibliche Konnektorelement, ein Rückschlagventil, vorzugsweise ein Schnabelventil, umfasst. Hierdurch wird ein Rückfluss von Fluid in den Milchbehälter verhindert und dadurch eine Kontamination der kostbaren Muttermilch verhindert.

Weiterhin liegt im Bereich der Erfindung auch ein Flüssigkeitsbehälter für die enterale Ernährung, insbesondere eine Milchflasche, umfassend eine solche Kappe, bei der die Kappe lösbar mit dem Flüssigkeitsbehälter, insbesondere mit der Milchflasche, verbunden ist oder fest mit dem Flüssigkeitsbehälter, insbesondere mit der Milchflasche, verbunden ist, vorzugsweise einteilig mit dem Flüssigkeitsbehälter, insbesondere der Milchflasche, ausgeführt ist.

Im Rahmen der Erfindung liegt ferner ein Set umfassend eine solche Kappe, wobei das Set einen Flüssigkeitsbehälter für die enterale Ernährung, insbesondere eine Milchflasche, umfasst. Dabei kann vorgesehen sein, dass das Set zudem eine Spritze mit einem männlichen Konnektorelement umfasst. Ferner kann vorgesehen sein, dass das Set zudem eine als ein Rohr oder als ein Schlauch ausgebildete Verlängerung mit einem ersten Ende, das mit dem weiblichen Konnektorelement der Kappe verbindbar ist, und einem zweiten Ende mit einem weiteren weiblichen Konnektorelement zum Verbinden mit einem männlichen Konnektorelement umfasst. Es kann ein Rohr oder einen flexibler Schlauch umfassen, das oder der an einem ersten Ende mit der Öffnung verbindbar oder verbunden ist und der am anderen Ende eine Vertiefung für ein männliches Konnektorelement umfasst, wobei vorzugsweise am anderen Ende ein Verschluss angeordnet ist, mit dem der Schlauch oder das Rohr abdeckbar oder abgedeckt ist. Hierdurch kann auch eine ungünstig positionierte Spritze über die Kappe mit einem für die Spritze schlecht oder gar nicht erreichbaren Milchbehälter verbunden werden.

Der Erfindung liegt die Erkenntnis zugrunde, dass es durch einen einfachen Adapter (eine Kappe) mit einer Vertiefung zur Aufnahme eines männlichen Konnektorelements gelingt, handelsübliche Milchflaschen für Muttermilch auch zur enteralen Ernährung von Frühgeborenen zu verwenden. Damit können mit Hilfe der erfindungsgemäßen Kappe die gleichen Milchflaschen, die zur Versorgung von Babys mit einem Sauger eingesetzt werden, auch zur Versorgung von Frühgeborenen bei enteraler Ernährung verwendet werden. Durch die antimikrobielle Oberfläche, beispielsweise einer Beschichtung, kann sichergestellt werden, dass es dabei zu keiner Kontamination der Muttermilch kommt.

Erfindungsgemäße Kappen für Milchflaschen bieten also das Sicherheitsmerkmal, dass eine Kontamination oder Verschmutzung mit Bakterien oder anderen Keimen nicht auftreten kann. Zudem wird eine sichere Verbindung zwischen einer Spritze und dem Milchbehälter sichergestellt, so dass keine Muttermilch verloren gehen kann. Konnektorelemente basierend auf dem ENLock-Verbindungssystem (konische Vertiefung als weibliches Konnektorelement mit Innengewinde und die konische Spitze einer Spritze als männliches Konnektorelement mit Außengewinde) verhindern die Aufnahme von Spitzen von Spritzen für intravenöse Behandlungen basierend auf dem Luer- oder Luerlock-System (bei denen das weibliche Konnektorelement ein Außengewinde umfasst und das männliche Konnektorelement von einem Rohrstück mit einem Innengewinde umgeben ist), so dass es zu keiner Verwechslung kommen kann und auch die Gefahr für eine Verbindung in falscher Richtung eliminiert wird. Die dichte Verbindung garantiert einen verlustfreien Durchgang der Muttermilch.

Die Kappe kann silberbeschichtet sein, um über längere Zeit gegen Bakterien oder Mikroorganismen wirksam zu sein. Die Kappe kann als Milchflaschenadapter oder Milchflaschenaufsatz verwendet werden. Sie kann erfindungsgemäß ein Belüftungsventil mit integriertem Bakterienfilter umfassen. Dadurch wird das Aufziehen von Flüssigkeiten aus flexiblen Milchbehältern (wie Beuteln) oder unflexiblen Milchbehältern (wie Spritzen, Aufnahmebehältern oder Babyflaschen) ermöglicht. Das Belüftungsventil ermöglicht das Einströmen oder Nachströmen von Luft und verhindert das Austreten von Flüssigkeit.

Ein integriertes Entenschnabel-Ventil kann einen unidirektionalen Fluss durch die Kappe ermöglichen, wobei ein Vorwärts-Fluss (aus dem Milchbehälter hinaus) möglich ist und eine Kontamination durch einen Rückfluss verhindert wird.

Da erfindungsgemäße Milchbehälter gegebenenfalls mit den Kappen gelegentlich in Kühlschränken aufbewahrt werden, können zumindest die Filter aus einem hydrophoben Material gefertigt sein, um eine Verstopfung oder Beeinträchtigung der Filter durch Kondenswasser zu verhindern.

Um eine Leckage des Inhalts erfindungsgemäßer Milchbehälter zu verhindern, wenn diese mit der Öffnung nach unten verwendet werden, kann erfindungsgemäß ein automatisches Verschlusssystem an der Kappe, insbesondere an deren bodenseitigen (dem Milchbehälter zugewandten) Ende vorgesehen sein.

Durch die Verwendung einer antimikrobiellen Beschichtung im Bereich der Öffnung, insbesondere in der Vertiefung (beispielsweise mit Silber oder Nano-Silber) können Mikroorganismen wie Bakterien und Pilze abgetötet werden. Zudem kann hierdurch die Anwendung von Desinfektionsmitteln reduziert werden. Da Desinfektionsmittel zu allergischen Reaktionen der Patienten, insbesondere bei Kleinkindern führen können, kann hierdurch eine zusätzliche Belastung der Patienten vermieden werden.

Eine Kronkorkenpassung sowie Foliensägezähne an der Innenseite der Kappe sind nicht erforderlich aber möglich. Die Kappe kann im Wesentlichen aus einem Spritzgussteil aus Kunststoff gefertigt werden. Ein Zusammensetzen, wie beispielsweise ein Verkleben mehrerer Einzelteile ist nicht notwendig.

Die Kappe wird als Verbindung zwischen einer Milchflasche und einer Spritze mit einem männlichen Konnektorelement eingesetzt und kann sowohl im Klinik- als auch im Home-Care-Bereich zum Einsatz kommen.

Im Folgenden werden Ausführungsbeispiele der Erfindung anhand von sieben schematisch dargestellten Figuren erläutert, ohne jedoch dabei die Erfindung zu beschränken. Dabei zeigt:
- Figur 1:: eine schematische perspektivische Ansicht einer erfindungsgemäßen Kappe;
- Figur 2:: eine schematische perspektivische geschnittene Ansicht einer erfindungsgemäßen Kappe mit eingesetzter Spitze einer Spritze;
- Figur 3:: eine schematische perspektivische Ansicht einer erfindungsgemäßen Kappe mit einer Verlängerung;
- Figur 4:: eine schematische Seitenansicht einer erfindungsgemäßen Kappe;
- Figur 5:: eine schematische perspektivische Ansicht einer erfindungsgemäßen Kappe mit noch nicht eingesetzter Spritze;
- Figur 6:: eine schematische perspektivische Ansicht einer erfindungsgemäßen Kappe mit noch nicht angeschlossener Milchflasche; und
- Figur 7:: eine schematische Querschnittansicht einer erfindungsgemäßen Kappe mit einem Entenschnabel-Ventil in der Öffnung.

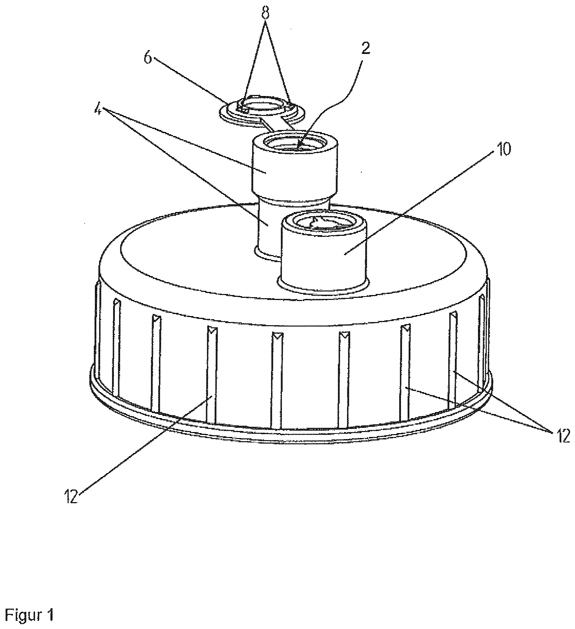

Figur 1 zeigt eine schematische perspektivische Ansicht einer erfindungsgemäßen Kappe. Die Kappe ist nach Art eines Schraubverschlusses für einen Milchbehälter (nicht gezeigt) mit einem Weithals aufgebaut. Sie kann als Schraubdeckel auf einen solchen Milchbehälter aufgeschraubt werden. Dazu umfasst die Kappe an der Unterseite ein Innengewinde (in Figur 1 nicht zu sehen).

In der Kappe ist eine durchgehende Öffnung oder Durchgangsöffnung 2 vorgesehen, die eine konische Vertiefung umfasst. Die konische Vertiefung wird durch ein, insbesondere außen zylindrisches, Rohr 4 mit konisch zusammenlaufendem Innendurchmesser realisiert, das in der Kappe angeordnet ist. Das Rohr 4 stellt ein weibliches Konnektorelement bereit. Die Öffnung 2 bzw. das weibliche Konnektorelement ist an dem oberen Ende durch einen Verschluss 6 verschließbar. Der Verschluss 6 ist über eine flexible Verbindung mit dem zylindrischen Rohr 4 verbunden, so dass der Verschluss 6 nicht verloren gehen kann. Das zylindrische Rohr 4 kann mit dem Verschluss 6 einteilig ausgeführt sein. Der Verschluss 6 umfasst eine lösbare Rastung 8, die in eine Gegenrastung am Rohr 4 greift, so dass der Verschluss 6 auf dem zylindrischen Rohr 4 lösbar befestigt werden kann. Der Verschluss 6 dient dazu, dass keine Verunreinigungen durch die Öffnung 2 durchtreten können, wenn keine Spritze (nicht gezeigt) mit der Öffnung 2 verbunden ist.

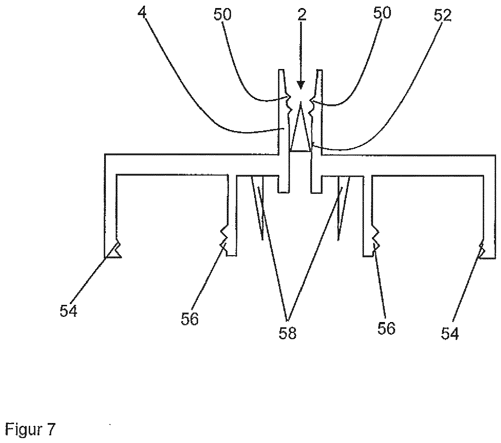

Die Kappe kann im Wesentlichen oder sogar vollständig aus Kunststoff bestehen und vorzugsweise durch ein Spritzgießverfahren hergestellt werden. In dem Rohr oder dem weiblichen Konnektorelement 4 ist ein Rückschlagventil 52, vorzugsweise ausgebildet als ein Entenschnabel-Ventil 52, angeordnet (siehe dazu Figur 7 und die zugehörige Beschreibung). Das Rückschlagventil 50 ist so angeordnet, dass die Entnahme von Milch aus dem Behälter möglich ist. Das Rückschlagventil 52 verhindert somit das Eintreten einer Flüssigkeit von oberhalb der Kappe durch die Öffnung 2 hindurch nach unten in den Behälter hinein. Bei angeschlossenem Milchbehälter und angeschlossener Spritze wird so ein Flüssigkeitsstrom von der Spritze in den Milchbehälter verhindert. Das integrierte Entenschnabel-Ventil erlaubt einen unidirektionalen Fluss durch die Kappe, wobei ein Vorwärts-Fluss (aus dem angeschlossenen Milchbehälter hinaus) möglich ist und eine Kontamination durch einen Rückfluss in den Milchbehälter verhindert wird.

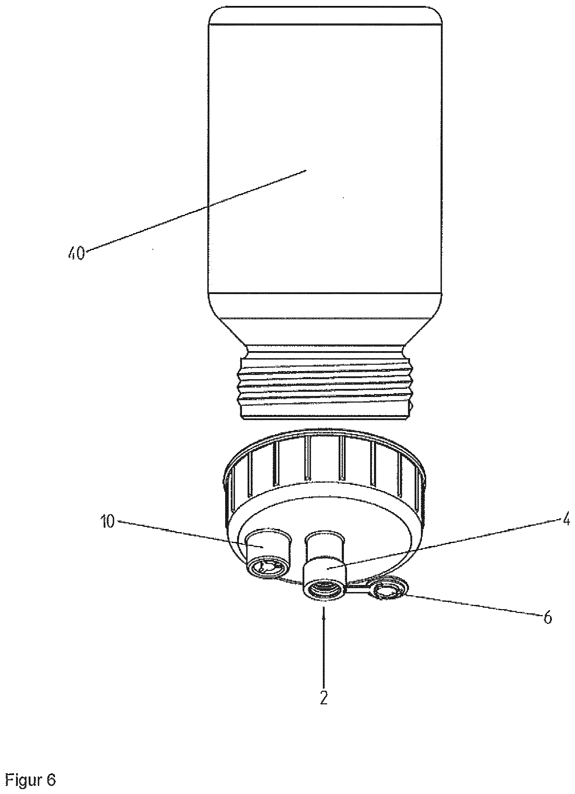

Die Geometrie der Öffnung 2 in der Kappe kann dafür sorgen, dass die gesamte Muttermilch aus einem angeschlossenen Milchbehälter 40 entnommen werden kann, wenn der Milchbehälter 40 kopfüber mit der Kappe verbunden ist (wie beispielsweise in Figur 6 gezeigt). Beispielsweise fluchtet die Unterseite des weiblichen Konnektorelements mit der Unterseite der Kappe.

Die Innenwände des Rohrs 4 bzw. des weiblichen Konnektors sind mit einer antibakteriellen Beschichtung aus Silber, beispielsweise aus Silber-Nano-Partikeln, beschichtet. Zudem oder alternativ kann die Beschichtung auch andere oder weitere pharmazeutisch aktive Substanzen umfassen, die zur Abtötung von Keimen, Pilzen und Mikroorganismen dienen. So kann verhindert werden, dass die Muttermilch bei der Entnahme mit einer Spritze durch die Öffnung 2 kontaminiert wird.

Auf der Oberseite oder in der Kappe ist auch ein Belüftungsventil 10 umfassend einen Filter zum Filtern von Mikroorganismen angeordnet. Über das Belüftungsventil 10 kann Luft von oberhalb der Kappe durch die Kappe nach unten hindurchtreten, wobei die Luft dabei durch den Filter gefiltert wird. So kann Luft von außen in einen angeschlossenen Milchbehälter einströmen, um einen Druckausgleich in dem Milchbehälter bei der Entnahme von Flüssigkeit aus dem Milchbehälter zu ermöglichen. Der Filter dient dazu, eine Verunreinigung und eine Kontamination der Muttermilch in dem Milchbehälter zu verhindern.

Die außenliegenden Seiten der Kappe sind allseitig mit vorspringenden Leisten 12 besetzt. Diese Leisten 12 dienen dazu, dass die Kappe zum Befestigen und Lösen von einem Milchbehälter leicht geschraubt werden kann, die Finger eines Anwenders also nicht so leicht abrutschen.

Die gezeigte Kappe ist ein erfindungsgemäßer Milchbehälterdeckel, der auf einem Milchbehälter aufschraubbar ist. Der Milchbehälterdeckel weist ein weibliches Konnektorelement auf, in dessen Öffnung ein männliches Konnektorelement einer Spritze zur Entnahme von Muttermilch eingesetzt werden kann. Das weibliche Konnektorelement wird im Wesentlichen durch das Rohr 4 bereitgestellt. Der Innendurchmesser und/oder die Gestalt des Rohrs 4 ist bzw. sind an die äußere Form des männlichen Konnektorelements der Spritze angepasst. Das männliche Konnektorelement der Spritze liegt, wenn es in die Öffnung 2 eingesetzt ist, dicht an den Innenwänden des Rohrs 4 an und schließt dadurch die Durchgangsöffnung 2 dicht ab. Die antimikrobielle Beschichtung, die zumindest auf den Innenwänden des Rohrs 4 angeordnet ist, verhindert eine Kontamination der Muttermilch. Solche Verbindungen sind für medizinische Zwecke besonders geeignet und ermöglichen schnelle Wechsel der Spritzen. In einer Ausgestaltung sind das weibliche Konnektorelement und/oder das männliche Konnektorelement ausgebildet als ein weibliches bzw. männliches ENLock-Konnektorelement oder PG-Lock-Konnektorelement. Das genannte PG-Lock- oder ENLock-Verbindungssystem hat den besonderen Vorteil, dass es nicht mit einer Verbindung für intravenöse Spritzen und Systeme, insbesondere basierend auf Luer oder Luer-Lock, verwechselbar oder kompatibel ist, so dass die Gefahr einer Fehlbedienung reduziert wird.

Beispielsweise weist ein ENLock-Verbindungssystem eine Schraubverbindung zum lösbaren Verbinden des männlichen mit dem weiblichen Konnektorelement auf. Aus Gründen einer vereinfachten Darstellung zeigen die Figuren das Verbindungssystem nicht als ein PG-Lock- oder als ein ENLock-Verbindungssystem sondern illustrieren es als einfache Schraubverbindung mit einem Gewindegang.

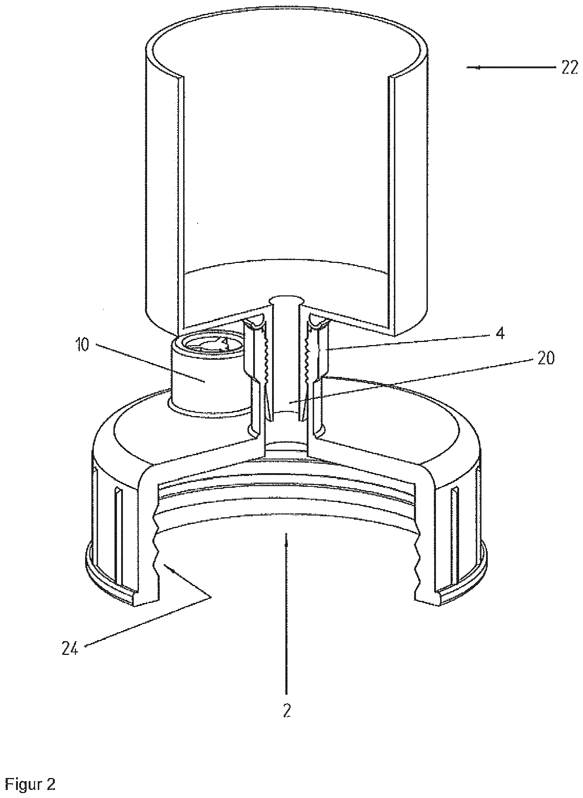

Figur 2 zeigt in einer perspektivischen teilgeschnittenen Ansicht eine erfindungsgemäße Kappe mit einem eingesetzten männlichen Konnektorelement 20, hier in Form einer eingesetzten Spitze 20 einer Spritze 22. Die Kappe umfasst ein Rohr 4, das eine Durchgangsöffnung 2 bereitstellt. Die Innenseite in dem Rohr 4 umfasst ein Innengewinde, so dass das männliche Konnektorelement 20 der Spritze 22, auf dem ein Außengewinde angeordnet ist, eingeschraubt werden kann. Hierdurch kann eine stabile Verbindung zwischen der Kappe und der Spritze 22 bereitgestellt werden. Die Innenseite des Rohrs 4 ist mit einer antimikrobiellen Beschichtung beschichtet.

Die Kappe umfasst ein Belüftungsventil 10 zur Belüftung einer mit der Kappe verbundenen oder verbindbaren Milchflasche (nicht gezeigt). Vorzugsweise sind das weibliche Konnektorelement und das männliche Konnektorelement ausgebildet als ein weibliches bzw. männliches PG-Lock- oder ENLock-Konnektorelement. Ein PG-Lock-Verbindungssystem ist dem Fachmann bekannt und liegt der ISO Arbeitsgruppe ISO/CD 80369-3 "Enterale und gastrische Anwendungen" als Entwurf vor und ist dort einsehbar. Ein ENLock-Verbindungssystem ist dem Fachmann bekannt und ist unter anderem in der WO 2010/115598 A1 beschrieben. Ferner liegt das ENLock-Verbindungssystem als Entwurf der ISO Arbeitsgruppe - TC210 / JWG4, PG 3 - Enteral Connectors - vor und ist einsehbar.

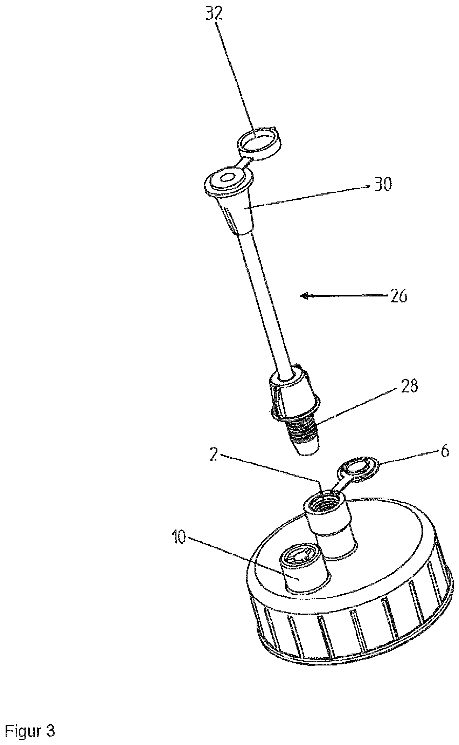

Unter bestimmten Bedingungen kann es sinnvoll sein, dass die Spritze nicht direkt mit der Öffnung 2 verbunden wird. Figur 3 zeigt hierzu eine schematische perspektivische Ansicht einer erfindungsgemäßen Kappe mit einer Verlängerung 26. Die Verlängerung 26 umfasst einen Adapter 28, der wie ein männliches Konnektorelement einer Spritze geformt ist. Der Adapter 28 kann daher mit der Öffnung 2 der Kappe verbunden werden, wenn er in das zylindrische Rohr 4 oder in das weibliche Konnektorelement gesteckt wird.

Am anderen Ende der Verlängerung 26 ist eine Aufnahme 30 angeordnet, deren Inneres wie das Innere des weiblichen Konnektorelements der Kappe ausgebildet ist.

Dadurch kann die Spritze mit dem männlichen Konnektorelement in die Aufnahme 30 der Verlängerung 26 eingesetzt werden und Muttermilch aus einem an der Kappe angeschlossenen Milchbehälter durch die Verlängerung 26 entnommen werden. Die Verlängerung 26 kann einen Kunststoffschlauch oder ein Kunststoffrohr umfassen. Grundsätzlich sind auch andere Materialien als Kunststoffe möglich und verwendbar.

An der Aufnahme 30 ist ein Verschluss 32 befestigt, mit dem die Verlängerung 26 abschließbar ist. Dazu ist der Verschluss 32 über eine flexible Schelle mit der Aufnahme 30 verbunden.

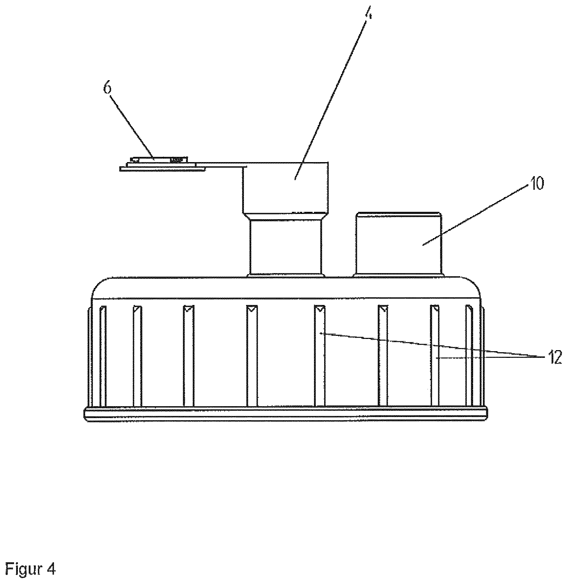

Figur 4 zeigt eine schematische Seitenansicht einer erfindungsgemäßen Kappe mit einem Rohr 4 als weiblichem Konnektorelement mit einer Öffnung (nicht zu sehen) als Durchführung durch die Kappe. Das Rohr 4 ist durch einen mit dem Rohr 4 verbundenen Verschluss 6 abdeckbar. Der Verschluss 6 besteht aus einem Kunststoff. Grundsätzlich kann die gesamte Kappe aus einem transparenten Kunststoff gefertigt sein, um eine visuelle Überprüfung des Inhalts eines angeschlossenen Milchbehälters zu ermöglichen. Eine weitere Durchführung oder Öffnung in der Kappe wird durch das Belüftungsventil 10 gebildet.

Ferner sind Leisten 12 am äußeren Umfang der Kappe als Eingriffe angeordnet, um die Kappe als Milchbehälterdeckel manuell auf einen Milchbehälter schrauben zu können. Das Rohr oder der weibliche Konnektor 4 ist auf der Innenseite vorzugsweise vollständig mit einer antimikrobiellen Schicht beschichtet.

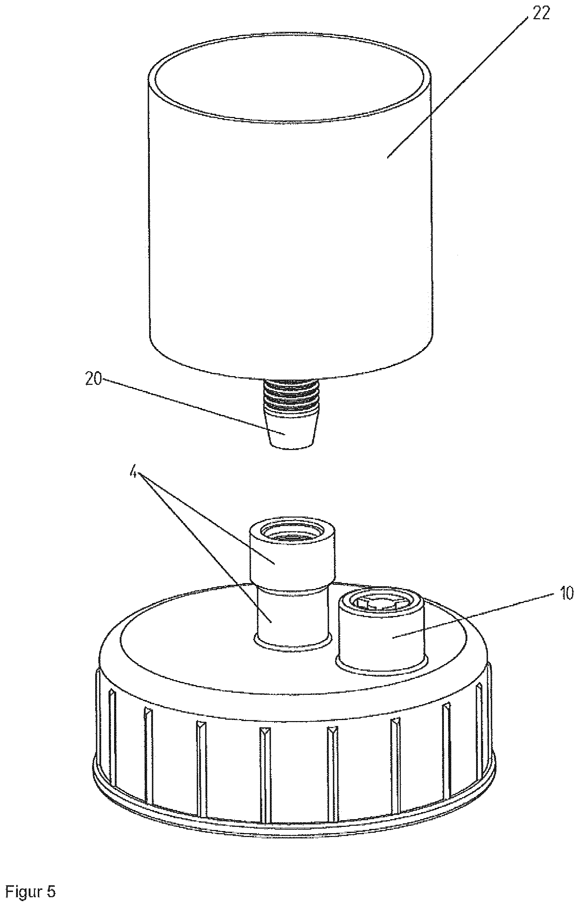

Figur 5 zeigt eine schematische perspektivische Ansicht einer erfindungsgemäßen Kappe mit einer noch nicht eingesetzten Spritze 22. Das männliche Konnektorelement 20 der Spritze 22 kann passgenau in die Öffnung der Kappe eingesetzt werden. Dazu steckt das männliche Konnektorelement 20, welches an seiner Außenseite abschnittsweise konisch ist, in dem Rohr oder weiblichen Konnektor 4, welcher bzw. weiches in seinem Inneren entsprechend abschnittsweise konisch ausgebildet ist, so dass die Spritze 22 dicht mit der Kappe verbunden ist. Um eine Kontamination der Spritze 22 und des durch die Durchgangsöffnung transportierten Mediums zu verhindern, sind die Innenwände des Rohrs oder weiblichen Konnektors 4 mit einer antibakteriellen Oberfläche versehen.

Die antibakterielle Schicht umfasst zu diesem Zweck Silberpartikel mit kleinem Durchmesser. Die Silberpartikel haben im Durchschnitt einen Durchmesser von weniger als 1 µm, um eine ausreichend große Silber-Oberfläche zu bilden. Neben dem Rohr 4 ist ein Belüftungsventil 10 angeordnet, durch das Luft in ein unten an die Kappe angeschlossenes Behältnis eindringen kann. Wenn das Behältnis keine flexiblen Wände aufweist, kann so für einen Druckausgleich gesorgt werden, wenn mit der Spritze ein flüssiges Medium aus dem Behältnis gezogen wird.

Figur 6 zeigt eine schematische perspektivische Ansicht einer erfindungsgemäßen Kappe und eine Milchflasche 40 mit einem Weithals, an die die Kappe anschließbar ist. Die Kappe und die Milchflasche 40 sind hier am Weithals über ein Gewinde miteinander verschraubbar.

Figur 7 zeigt eine schematische Querschnittansicht einer erfindungsgemäßen Kappe. Die Kappe umfasst eine zentrale Öffnung 2, die als Durchgang in einem Rohr 4 ausgebildet ist. Das Rohr 4 stellt das weibliche Konnektorelement bereit. Das obere Ende des Rohrs 4 ist nach unten konisch zusammenlaufend ausgebildet und weist im Inneren ein Innengewinde 50 auf. Das Rohr 4 ragt in Figur 7 nach oben über die Deckelfläche der Kappe hinaus. Nach unten hin schließt das Rohr 4 bündig mit der Kappe ab.

Im Inneren der Rohrs 4 ist ferner ein Entenschnabel-Ventil 52 angeordnet. Das Ventil 52 kann durch das Ansaugen mittels einer angeschlossenen Spritze (nicht gezeigt) geöffnet werden kann. Durch die Einwegfunktion des Ventils 52 wird ein Rückfluss in den Behälter verhindert. Im nicht angeschlossen Zustand der Spritze ist das Ventil 52 geschlossen.

Auf der Unterseite der Kappe sind zwei Befestigungsmittel 54, 56 angeordnet. Das äußere Befestigungsmittel 54 ist ein Innengewinde 54 zur Befestigung eines Milchbehälters mit einem Weithals mit Außengewinde. Das Innere Befestigungsmittel 56 ist ein Außengewinde 56 zur Befestigung eines Milchbehälters mit einem Dünnhals mit Innengewinde. Des Weiteren sind an der Unterseite der Kappe Scheiden 58 angeordnet, die eine Schutzfolie aufschneiden können, mit der der Milchbehälter (nicht gezeigt) verschlossen sein können.

Die Kappe ist so für verschiedene Milchbehälter geeignet. Beispielsweise für solche mit Weithals und mit Dünnhals sowie auch für solche, die mit einer Schutzfolie abgedeckt sind. Variable Mehrzweckanschlüsse erlauben die Verwendung mit verschiedenen Milchbehältern. Vorzugsweise ist die Kappe ein Mehrzweckkonnektor zur enteralen Applikation, wie er in der Anmeldung WO 2008/028 582 A1 beschrieben ist.

Die in der voran stehenden Beschreibung, sowie den Ansprüchen, Figuren und Ausführungsbeispielen offenbarten Merkmale der Erfindung können sowohl einzeln, als auch in jeder beliebigen Kombination für die Verwirklichung der Erfindung in ihren verschiedenen Ausführungsformen wesentlich sein und gegebenenfalls und miteinander kombiniert werden.

### Bezugszeichenliste

- 2: Öffnung
- 4: Rohr oder weibliches Konnektorelement
- 6, 32: Verschluss
- 8: Rastung
- 10: Belüftungsventil
- 12: Leiste
- 20: Spitze / männliches Konnektorelement
- 22: Spritze
- 24: Gewinde
- 26: Verlängerung
- 28: Adapter
- 30: Aufnahme
- 40: Milchflasche
- 50: Innengewinde
- 52: Entenschnabel-Ventil
- 54, 56: Gewinde / Befestigungsmittel
- 58: Schneide

## Patentansprüche

1. Kappe für einen Behälter zur Aufnahme einer Flüssigkeit für die enterale Ernährung, insbesondere für einen Milchbehälter (40), umfassend eine Halterung (54, 56) zum Befestigen der Kappe an dem Behälter (40) und ein als Durchgangsöffnung in der Kappe angeordnetes weibliches Konnektorelement zum Verbinden mit einem männlichen Konnektorelement (20), wobei die Kappe einen fluiddichten Belüftungskanal aufweist , **dadurch gekennzeichnet, dass** wenigstens das weibliche Konnektorelement zumindest abschnittsweise eine antimikrobielle Oberfläche aufweist,welche durch eine antimikrobielle Beschichtung bereitgestellt ist.

2. Kappe nach Anspruch 1, **dadurch gekennzeichnet, dass** die Halterung (54, 56) als ein Weithalsflaschenadapter (54), ein Schmalhalsflaschenadapter (56) und/oder eine Steckverbindung ausgebildet ist.

3. Kappe nach einem der vorangehenden Ansprüche, **dadurch gekennzeichnet, dass** das weibliche Konnektorelement zumindest abschnittsweise konisch ist und in dem weiblichen Konnektorelement ein zumindest abschnittsweise konisches, männliches Konnektorelement (20) aufnehmbar ist.

4. Kappe nach einem der vorangehenden Ansprüche, **dadurch gekennzeichnet, dass** die antimikrobielle Oberfläche Silber umfasst.

5. Kappe nach einem der vorangehenden Ansprüche, **dadurch gekennzeichnet, dass** der fluiddichte Belüftungskanal ein Belüftungsventil (10) und/oder einen Belüftungsfilter, insbesondere einen Bakterienfilter, umfasst.

6. Kappe nachAnspruch 5, **dadurch gekennzeichnet, dass** das Belüftungsventil (10) und/oder der Belüftungsfilter zumindest bereichsweise eine hydrophobe Oberfläche aufweist bzw. aufweisen.

7. Kappe nach einem der vorangehenden Ansprüche, **dadurch gekennzeichnet, dass** die Kappe, vorzugsweise das weibliche Konnektorelement, eine Befestigungseinrichtung (50) zur Befestigung des männlichen Konnektorelements (20) umfasst.

8. Kappe nach vorangehendem Anspruch, **dadurch gekennzeichnet, dass** die Befestigungseinrichtung (50) als ein Drehverriegelungselement, ein Bajonett-Verschluss oder ein Gewinde (50) ausgeführt ist, wobei die Befestigungseinrichtung (50) lösbar mit dem männlichen Konnektorelement (20) verbindbar ist.

9. Kappe nach vorangehendem Anspruch, **dadurch gekennzeichnet, dass** das Gewinde als ein Innengewinde (50) in dem weiblichen Konnektorelement ausgebildet ist zum Befestigen eines männlichen Konnektorelements (20) mit einem Außengewinde.

10. Kappe nach einem der vorangehenden Ansprüche, **dadurch gekennzeichnet, dass** sich das weibliche Konnektorelement, vorzugsweise zentrisch, durch die Kappe erstreckt und/oder über eine Deckelfläche der Kappe hinaus erstreckt und vorzugsweise durch einen Verschluss (6) abdeckbar ist.

11. Kappe nach einem der vorangehenden Ansprüche, **dadurch gekennzeichnet, dass** dem weiblichen Konnektorelement ein Rückschlagventil, vorzugsweise ausgebildet als ein Schnabelventil (58), zugeordnet ist.

12. Flüssigkeitsbehälter (40) für die enterale Ernährung umfassend eine Kappe nach einem der vorangehenden Ansprüche, **dadurch gekennzeichnet, dass** die Kappe lösbar mit dem Flüssigkeitsbehälter (40) verbunden ist oder fest mit dem Flüssigkeitsbehälter (40) verbunden ist, vorzugsweise einteilig mit dem Flüssigkeitsbehälter (40) ausgeführt ist.

13. Set umfassend einen Flüssigkeitsbehälter (40) nach Anspruch 12 und eine Kappe nach einem der Ansprüche 1 bis 11.

14. Set nach Anspruch 13 **gekennzeichnet durch** eine Spritze (22) mit einem männlichen Konnektorelement (20) und/oder eine als ein Rohr oder als ein Schlauch (26) ausgebildete Verlängerung mit einem ersten Ende, das mit dem weiblichen Konnektorelement der Kappe verbindbar ist, und einem zweiten Ende mit einem weiteren weiblichen Konnektorelement zum Verbinden mit einem männlichen Konnektorelement (20).

## Claims

1. Cap for a container for accommodating a liquid for enteral nutrition, in particular for a milk container (40), comprising a holder (54, 56) for fastening the cap on the container (40) and also comprising a female connector element, which is arranged in the form of a through-opening in the cap and is intended for connecting to a male connector element (20), wherein the cap has a fluid-tight ventilating channel, **characterized in that** at least the female connector element, at least in part, has an antimicrobial surface, which is provided by an antimicrobial coating.

2. Cap according to Claim 1, **characterized in that** the holder (54, 56) is designed in the form of a wideneck-bottle adapter (54), a narrowneck-bottle adapter (56) and/or a plug-in connection.

3. Cap according to one of the preceding claims, **characterized in that** the female connector element, at least in part, is conical, and an at least partially conical male connector element (20) can be accommodated in the female connector element.

4. Cap according to one of the preceding claims, **characterized in that** the antimicrobial surface comprises silver.

5. Cap according to one of the preceding claims, **characterized in that** the fluid-tight ventilating channel comprises a ventilating valve (10) and/or a ventilating filter, in particular a bacteria filter.

6. Cap according to Claim 5, **characterized in that** the ventilating valve (10) and/or the ventilating filter have/has, at least in certain regions, a hydrophobic surface.

7. Cap according to one of the preceding claims, **characterized in that** the cap, preferably the female connector element, comprises a fastening device (50) for fastening the male connector element (20).

8. Cap according to the preceding claim, **characterized in that** the fastening device (50) is configured in the form of a rotary locking element, a bayonet closure or a thread (50), wherein the fastening device (50) can be connected in a releaseable manner to the male connector element (20).

9. Cap according to the preceding claim, **characterized in that** the thread is designed in the form of an internal thread (50) in the female connector element, for fastening a male connector element (20) with an external thread.

10. Cap according to one of the preceding claims, **characterized in that** the female connector element extends, preferably centrally, through the cap and/or extends beyond a top surface of the cap and can be covered preferably by a closure (6).

11. Cap according to one of the preceding claims, **characterized in that** the female connector element is assigned a nonreturn valve, preferably designed in the form of a duckbill valve (58).

12. Liquid container (40) for enteral nutrition, comprising a cap according to one of the preceding claims, **characterized in that** the cap is connected in a releasable manner to the liquid container (40) or is fixed to the liquid container (40), preferably is configured in one piece with the liquid container (40).

13. Set comprising a liquid container (40) according to Claim 12 and a cap according to one of Claims 1 to 11.

14. Set according to Claim 13, **characterized by** a syringe (22) having a male connector element (20) and/or by an extension which is in the form of a tube or of a hose (26) and has a first end, which can be connected to the female connector element of the cap, and a second end, which has a further female connector element for connecting to a male connector element (20).

## Revendications

1. Capuchon pour un récipient destiné à contenir un liquide pour l'alimentation entérale, en particulier pour une bouteille de lait (40), comprenant un support (54, 56) pour la fixation du capuchon sur le récipient (40) et un élément de connecteur femelle disposé dans le capuchon en tant qu'ouverture de passage à relier à un élément de connecteur mâle, dans lequel le capuchon présente un canal d'aération étanche au fluide, **caractérisé en ce qu'**au moins l'élément de connecteur femelle présente au moins localement une surface antimicrobienne, qui est constituée par un revêtement antimicrobien.

2. Capuchon selon la revendication 1, **caractérisé en ce que** le support (54, 56) est réalisé sous la forme d'un adaptateur de bouteille à large col (54), d'un adaptateur de bouteille à col étroit (56) et/ou d'un connecteur de raccordement.

3. Capuchon selon l'une quelconque des revendications précédentes, **caractérisé en ce que** l'élément de connecteur femelle est au moins localement conique et un élément de connecteur mâle (20) au moins localement conique peut être agencé dans l'élément de connecteur femelle.

4. Capuchon selon l'une quelconque des revendications précédentes, **caractérisé en ce que** la surface antimicrobienne contient de l'argent.

5. Capuchon selon l'une quelconque des revendications précédentes, **caractérisé en ce que** le canal d'aération étanche au fluide comprend une soupape d'aération (10) et/ou un filtre d'aération, en particulier un filtre à bactéries.

6. Capuchon selon la revendication 5, **caractérisé en ce que** la soupape d'aération (10) et/ou le filtre d'aération présente(nt) au moins localement une surface hydrophobe.

7. Capuchon selon l'une quelconque des revendications précédentes, **caractérisé en ce que** le capuchon, de préférence l'élément de connecteur femelle, comprend un dispositif de fixation (50) pour la fixation de l'élément de connecteur mâle (20).

8. Capuchon selon la revendication précédente, **caractérisé en ce que** le dispositif de fixation (50) est réalisé en forme d'élément de verrouillage rotatif, de fermeture à baïonnette ou de filet (50), dans lequel le dispositif de fixation (50) peut être assemblé de façon démontable à l'élément de connecteur mâle (20).

9. Capuchon selon la revendication précédente, **caractérisé en ce que** le filet est réalisé en forme de filet intérieur (50) dans l'élément de connecteur femelle pour la fixation d'un élément de connecteur mâle (20) avec un filet extérieur.

10. Capuchon selon l'une quelconque des revendications précédentes, **caractérisé en ce que** l'élément de connecteur femelle s'étend, de préférence au centre, à travers le capuchon et/ou s'étend au-delà d'une face de couvercle du capuchon et peut de préférence être couvert par une fermeture (6).

11. Capuchon selon l'une quelconque des revendications précédentes, **caractérisé en ce qu'**un clapet antiretour, de préférence réalisé en forme de soupape à bec de canard (58), est associé à l'élément de connecteur femelle.

12. Récipient pour liquide (40) destiné à l'alimentation entérale, comprenant un capuchon selon l'une quelconque des revendications précédentes, **caractérisé en ce que** le capuchon est assemblé de façon séparable au récipient pour liquide (40) ou est solidaire du récipient pour liquide (40), et est de préférence réalisé d'une seule pièce avec le récipient pour liquide (40).

13. Set comprenant un récipient pour liquide (40) selon la revendication 12 et un capuchon selon l'une quelconque des revendications 1 à 11.

14. Set selon la revendication 13, **caractérisé par** une seringue (22) avec un élément de connecteur mâle (20) et/ou un prolongement réalisé en forme de tube ou de tuyau souple (26) avec une première extrémité, qui peut être raccordée à l'élément de connecteur femelle du capuchon, et avec une deuxième extrémité avec un autre élément de connecteur femelle à raccorder à un élément de connecteur mâle (20).
